# EUROPÄISCHE PATENTANMELDUNG

(11) **EP 0 864 325 A2**
(43) Veröffentlichungstag der Anmeldung: **16.09.1998**
(21) Anmeldenummer: 98104001.7
(22) Anmeldetag: 06.03.1998
(51) Int. Cl.: A61K 9/22, A61K 9/52, A61K 31/485, A61K 31/135

(54) **Feste, mindestens zweiphasige Zubereitungsformen eines Opioid-Analgeticums mit verzögerter Freisetzung**

(30) Priorität: 12.03.1997 DE 19710008
(71) Anmelder: BASF AKTIENGESELLSCHAFT, 67056 Ludwigshafen (DE)
(72) Erfinder: Berndl, Gunther, Dr., 67273 Herxheim (DE); Rosenberg, Joerg, Dr., 67158 Ellerstadt (DE); Breitenbach, Jörg, Dr., 68199 Mannheim (DE)

(57) **Zusammenfassung**

Feste, mindestens zweiphasige Zubereitungsformen mindestens eines Opioid-Analgeticums in einer Matrix, wobei jede der beiden Phasen mindestens ein Opioid enthält, mit einer in-vitro-Freisetzungsrate nach dem Ph.Eur. Paddle Modell von mehr als 50 Gew.-% Wirkstoff nach einer Stunde.

## Beschreibung

Die vorliegende Erfindung betrifft feste, mindestens zweiphasige Zubereitungsformen mindestens eines Opioid-Analgeticums, wobei jede der beiden Phasen mindestens ein Opioid enthält, mit einer in-vitro-Freisetzungsrate, bestimmt nach dem Ph.Eur.Paddle-Modell, von mehr als 50 Gew.-% nach einer Stunde. Weiterhin betrifft die Erfindung ein Verfahren zur Herstellung solcher Zubereitungsformen.

Bisher bekannte Opioid-Arzneiformen setzen den Wirkstoff entweder unverzögert oder klassisch retardiert frei. Solche klassisch retardierten Formen weisen entweder einen retardierend wirkenden Überzug auf oder enthalten eine einheitliche Phase des Wirkstoffs in einer Hilfsstoffmatrix. Entsprechend werden einstufige Freisetzungsprofile erzielt. Nachteilig an einer klassischen Retardierung ist jedoch, daß die Freisetzung des Wirkstoffs relativ langsam einsetzt, was auch ein verzögertes Einsetzen der analgetischen Wirkung zur Folge hat. Nachteilig an einer Retardierung mit Hilfe eines Überzugs ist auch, daß ein sogenanntes "Dose-Dumping" auftreten kann, wenn der Überzug im Verdauungstrakt durch die Peristaltik beschädigt wird. Dabei kann es zu einer so großen Initialdosierung kommen, daß der therapeutische Bereich verlassen wird.

So werden in der EP-A 271 193 Hydromorphon-Formulierungen für eine zweimal tägliche Anwendung beschrieben, die den Wirkstoff in einer Matrix enthalten, und bei denen nach einer Stunde bis zu 42,5 Gew.-% des Wirkstoffes freigesetzt sind. Solche Formulierungen bestehen aus einer einheitlichen Phase.

In der EP-A 543 541 werden Zubereitungen des Wirkstoffs Flutamid beschrieben, die als äussere Hülle eine schnell freisetzende Phase aufweisen und einen Kern, der einen retardierenden Überzug aufweist.

Aufgabe der vorliegenden Erfindung war es, Arzneiformen zu finden, die gleichzeitig ein schnelles Anfluten des Wirkstoffs zur schnellen Reduktion starker Schmerzen und eine retardierte Freisetzung zur langandauernden Schmerzbekämpfung erlauben.

Demgemäß wurden Darreichungsformen gefunden, die eine mindestens biphasische Freisetzung des Wirkstoffs aufweisen. Die festen, oral zu verabreichenden, mindestens zweiphasigen Arzneiformen, wobei jede Phase mindestens ein Opioid-Analgeticum umfasst, zeigen eine in-vitro-Freisetzungsrate , gemessen nach dem Ph. Eur. Paddle-Modell, von mehr als 50 % des Wirkstoffs nach einer Stunde. Bevorzugt sind Arzneiformen mit einer Freisetzungsrate von 55 bis zu 80 % nach einer Stunde, bis zu 85% nach zwei Stunden, 60 bis 95% nach vier Stunden und zwischen 65 und 100% nach acht Stunden. Besonders bevorzugt sind Freisetzungsraten von 56 bis 70 % in der ersten Stunde. Die Arzneiformen sind vor allem für eine zweimal tägliche Applikation vorgesehen. Wenn nicht anders erwähnt , werden die in-vitro-Freisetzungsraten nach dem Ph. Eur. Paddle Modell bei 100 U/min in 0,1 N HCl bei 37°C und UV-Detektion bei 270 nm bestimmt.

Opioid-Analgetica im Sinne dieser Erfindung sind:

Morphin, Codein, Ethylmorphin, Dihydrocodein, Hydromorphon, Oxycodon, Hydrocodon, Thebacon,Levorphanol, Pethidin, Ketobemidon, Levomethadon, Normethadon, Fenpipramid, Dextromoramid, Clofenadol, Pentazocin, Tilidin, Naloxon, Piritramid, Fentanyl, Nefopam, Tramadol, Buprenorphin oder deren physiologisch verträgliche Salze.

Bevorzugte Opioide sind:

Codein, Dihydrocodein, Hydrocodon, Hydromorphon, Morphin, Dihydromorphin, Oxymorphon, Tramadol oder deren Mischungen. Ganz besonders bevorzugte Opioide sind ausgewählt aus der Gruppe Hydromorphon, Morphin, Tramadol oder deren physiologisch verträglichen Salzen.

Die beanspruchten Arzneiformen enthalten die Opioid-Wirkstoffe in therapeutisch wirksamen Mengen. Im Falle des Tramadols werden 50 bis 1000 mg Tramadolhydrochlorid pro Arzneiform eingesetzt, bevorzugt 100 bis 600 mg. Daran sind bevorzugt 5 - 50 Gew.-% in der IR-Phase, 50 bis 95 Gew.-% in der SR-Phase enthalten.

Im Falle des Morphins oder dessen physiologisch verträglicher Salze wie Morphinsulfat oder Morphinhydrochlorid werden 10 mg, 30 mg, 60 mg, 100 mg, 200 mg oder 500 mg Wirkstoff pro Arzneiform eingesetzt, wovon bevorzugt 1 bis 20 Gew.-% in der IR-Phase und 80 bis 99 Gew.-% in der SR-Phase enthalten sind.

Im Falle des Hydromorphons enthalten die Arzneiformen 1 bis 150 mg, bevorzugt 2 bis 60 mg, insbesondere 2 bis 30 mg, wovon bevorzugt 1 bis 15 Gew.-% in der IR-Phase und 85 - 99 Gew.-% in der SR-Phase enthalten sind.

Die Arzneiformen bestehen aus mindestens zwei Phasen, von denen jede Phase mindestens ein Opioid in einer Matrix enthält. Die Opioide können gleich oder verschieden sein. Die verschiedenen Phasen weisen jeweils ein unterschiedliches Freisetzungsprofil auf, wobei eine Phase schnell freisetzend ist (IR-Phase; Instant Release) und ein schnelles Einsetzen der analgetischen Wirkung erzielen soll, während die andere Phase retardiert ist (SR-Phase, Sustained Release), um zusätzlich eine langanhaltende Wirkung zu erreichen.

Die Steuerung der Freisetzung kann jeweils durch die Zusammensetzung der Matrix der einzelnen Phasen erreicht werden. Diese Matrix kann aus niedermolekularen Hilfsstoffen und/oder polymeren Bindemitteln bestehen. Die Steuerung der Freisetzung erfolgt nicht durch einen retardierenden Überzug.

Schnellfreisetzende Matrices können beispielsweise durch folgende Zusammensetzungen an Hilfsstoffen erhalten werden:
a) als polymere Bindemittel kommen synthetische oder halbsynthetische Polymere wie wasserlösliche Homo- oder Copolymere des N-Vinylpyrrolidons (NVP) mit K-Werten nach Fikentscher bis 30 in Betracht, wobei als Comonomere Vinylester, insbesondere Vinylacetat, bevorzugt sind, weiterhin Hydroxyalkylcellulosen wie beispielsweise die Hydroxypropylcellulose, Alkylcellulosen wie beispielsweise Ethylcellulose, ebenso wie Polyethylenglykole, bevorzugt solche mit Molekulargewichten von 1000 bis 20000. Weiterhin eignen sich Methacrylsäure-Copolymere (Eudragit-Typen) oder Polyvinylalkohol. Als Bindemittel eignen sich auch natürliche Polymere wie Stärke oder auch Stärkederivate wie beispielsweise Hydroxyethylstärke, oxidierte Stärken oder vorverkleisterte Stärken.
b) als Füllstoffe eignen sich Zucker, beispielsweise Lactose, Glucose oder Saccharose, Zuckeralkohole wie beispielsweise Maltit, Mannit, Sorbit, Xylit und Isomalt, ebenso abgebaute Stärken wie Dextrine, insbesondere Maltodextrin. Weiterhin eignen sich mikrokristalline Cellulose oder anorganische Salze wie beispielsweise Calciumphosphat, Dicalciumphosphat oder Natriumchlorid sowie gegebenenfalls auch Alkalicarbonate wie beispielsweise Na₂CO₃.
c) als Sprengmittel eignen sich Natriumstärkeglycolat, quervernetztes Polyvinylpyrrolidon, quervernetzte Carboxymethylcellulose, Bentonite, Alginate oder Croscarmellose.
d) als wasserlösliche Netzmittel oder wasserlösliche Tenside eignen sich Natriumlaurylsulfat, Sorbitanfettsäureester, Salze von Gallensäuren und ähnliche pharmazeutisch akzeptable oberflächenaktive Substanzen.
e) weitere pharmazeutische zusatzstoffe wie Farb- und/oder Aromastoffe.

Die verschiedenen Komponenten können in der Matrix in den folgenden Mengen eingesetzt werden:
a) 0 bis 80 Gew.-%
b) 0 bis 99 Gew.-%
c) 1 bis 8 Gew.-%
d) 0,1 bis 10 Gew.-%
e) 0,1 bis 5 Gew.-%

Die Mengenangaben beziehen sich auf die Gesamtmenge der Hilfsstoffe (a) bis (e), wobei die Mengen an a) und b) jeweils so gewählt werden, daß sich die Gesamtmenge von 100 Gew.-% ergibt.

Die retardierte Phase kann folgende Inhaltsstoffe enthalten:

Als polymere Bindemittel kommen in Betracht:
A) lösliche Homo- und Copolymere des NVP mit K-Werten von 10 bis 100, wobei als Comonomere Vinylester wie Vinylpropionat oder insbesondere Vinylacetat bevorzugt sind, weiterhin Polyvinylalkohol, Polyvinylacetat, teilverseiftes Polyvinylacetat,
B) Alkylcellulosen wie Methylcellulose oder Ethylcellulose, Hydroxyalkylcellulosen wie Hydroxypropylcellulose, Hydroxyethylcellulose sowie Hydroxypropylmethylcellulose, weiterhin Celluloseester wie Celluloseacetat, Celluloseacetatpropionat, Celluloseacetatphthalat, Hydroxypropylmethylcellulosephthalat, Hydroxypropylmethylcelluloseacetatphthalat sowie unvernetzte Carboxymethylcellulose,
C) Methacrylsäurecopolymere, wobei als Comonomere Ethylacrylat bevorzugt ist, Aminoalkylmethacrylat-Copolymere.

Es können auch Mischung der Polymere A), B) und/oder C) eingesetzt werden, beispielsweise Mischungen aus 0 bis 90 Gew.-% A), 10 bis 70 Gew.-% B) und 0 bis 20 Gew.-% C).

Weiterhin können die jeweiligen Phasen zusätzliche pharmazeutische Hilfsstoffe wie Gleitmittel, Geschmacksverbesserer, Emulgatoren etc. enthalten.

Die Arzneiformen können auch mehr als zwei opioidhaltige Phasen enthalten, wenn eine stärkere Staffelung der retardierten Freisetzung erzielt werden soll. Weiterhin können die Arzneiformen zusätzlich nicht-opioidhaltige Phasen enthalten, welche andere Analgetica wie beispielsweise nichtsteroidale, antiinflammatorische Analgetica enthalten können.

Bevorzugt liegen die erfindungsgemäßen Arzneiformen als offene Mehrschicht-Tabletten vor, in denen jeweils eine Phase eine Schicht bildet. Die Arzneiformen können auch als geschlossene Manteltabletten vorliegen, in denen die schnellfreisetzende Phase den äusseren Mantel bildet, während der Kern aus einer oder mehreren retardierten Phasen gebildet wird. Als mehrphasige Arzneiformen kommen auch sogenannte Punkttabletten in Betracht (Bull-eye-Tabletten) oder multipartikuläre Formen, in denen einzelne Granulate, Pellets oder andere Partikel, die der retardierten Phase entsprechen in eine schnellfreisetzende Phase eingebettet sind.

Zur Herstellung der erfindungsgemässen Arzneiformen eignen sich unter anderem konventionelle Tablettierungsverfahren, beispielsweise Verfahren bei denen die jeweils einzelnen Phasen in Form von Granulaten oder Pellets zusammen verpresst werden. Dabei bleiben die einzelnen Phasen mit ihrem unterschiedlichen Freisetzungsverhalten erhalten. Auf diese Weise lassen sich Ein- oder Mehrschicht-Tabletten erhalten.

Bevorzugt erfolgt die Herstellung der erfindungsgemässen Arzneiformen durch ein Schmelzextrusionsverfahren, wobei die Wirkstoffe mit den Hilfsstoffen in der Schmelze vermischt, durch eine oder mehrere Düsen oder Lochplatten extrudiert und anschliessend die noch thermoplastischen Massen der Formgebung unterworfen werden. Dieses Verfahren wird bevorzugt in Abwesenheit von Lösungsmitteln durchgeführt. Zur Herstellung von offenen oder geschlossenen Manteltabletten eignet sich vor allem das Verfahren der Coextrusion, bei dem die für die einzelnen Phasen vorgesehenen Zusammensetzungen separat aufgeschmolzen und extrudiert werden, worauf die noch plastischen Stränge, Bänder oder Schichten zusammengeführt und verformt werden. Ein solches Coextrusionsverfahren ist beispielsweise in der DE 195 39 361 beschrieben. Wahlweise können Mehrschichttabletten auch durch Kombination von Schmelzen in einem Spritzgussverfahren erfolgen, wobei in der Form sukzessive Schichten aufgebracht werden, oder durch 2-Komponenten-Spritzguss.

Die Herstellung erfolgt bevorzugt in Doppelschneckenextrudern mit oder ohne Knetelementen.

Weiterhin ist es möglich eine der Phasen in fester Form in eine Schmelze oder eine noch plastische Masse der anderen Phase einzubetten. Vorraussetzung dafür ist, daß sich die feste Form nicht in der Schmelze löst, was durch geeignete Wahl der Hilfsstoffe erreicht werden kann, oder durch Umhüllen oder Bepudern der einen Matrix vor Einbettung in die andere.

Nach dem erfindungsgemässen Verfahren herstellbare feste, mindestens zweiphasige Arzneiformen sind insbesondere Tabletten, vorzugeweise Oblongtabletten, Dragees, Pastillen oder Pellets. Die Arzneiformen könne auch mit einem geschmacksabdeckenden Überzug versehen werden.

Erfindungsgemäss werden Opioid-Arzneiformen erhalten, die aufgrund ihrer Zusammensetzung aus einer schnellfreisetzenden Phase und mindestens einer retardierten Phase eine für starke Analgetica besonders wünschenwerte Freisetzungskinetik aufweisen. Mit Hilfe des erfindungsgemässen Verfahrens lassen sich solche Formen besonders einfach und zuverlässig herstellen.

Durch Anwendung der erfindungsgemäßen Darreichungsformen wird nicht nur ein schnelles Einsetzen der schmerzstillenden Wirkung erzielt, sondern auch eine länger anhaltende Schmerzbekämpfung. Es war überraschend, dass auch nach 5 h Plasmaspiegel im schmerzrelevanten Bereich erzielt werden konnten.

Im Falle des Tramadols werden nach 5 Stunden unter Fasten-Bedingungen nach einmaliger Applikation Blutplasmaspiegel von 90 bis 200 mg/ml, bevorzugt 100 bis 180 mg/ml, besonders bevorzugt 110 bis 165 mg/ml erzielt.

Die nachstehend Beispielen genannten Zusammensetzungen werden bevorzugt in einem Doppelschneckenextruder ZSK 30 der Firma Werner & Pfleiderer bei je einem Durchsatz von 2 kg/Stunde coextrudiert. Die Formgebung des noch plastischen Extrudats erfolgt, wie in der EP-A 240 906 beschrieben, durch Kalandrierung.

### Formulierung 1:

### Extruder 1:

1 kg einer Mischung aus 40 Gew.-% Tramadol-HCl, 15 Gew.-% Ethylcellulose (Ethocel N100), 35 Gew.-% Isomalt, 7 Gew.-% Polyvinylpyrrolidon K30 und 3 Gew.-% hydriertes Rizinusöl (Cutina HR) wurden in einem Doppelschneckenextruder vermischt.
Temperatur der Schüsse: 44, 80, 121, 99, 92, 82 °C
Düse: 80 °C

### Extruder 2:

1 kg einer Mischung aus 35 Gew.-% Tramadol-HCl, 34 Gew.-% Hydroxypropylmethylcellulose (Methocel K100M) und 31 Gew.-% Hydroxypropylcellulose (Klucel EF) wurden in einem Doppelschneckenextruder vermischt.
Temperatur der Schüsse: 44, 80, 121, 99, 92, 82°C
Düse: 80 °C

Die Arzneiformen wiesen folgende in-vitro-Freisetzung auf.

| Zeit in Minuten | Freigesetzter Anteil Wirkstoff in Gew.-% |
|---|---|
| 60 | 71,9 |
| 120 | 81,9 |
| 180 | 87,9 |
| 240 | 90,9 |
| 300 | 95,6 |
| 360 | 97,0 |
| 420 | 97,5 |
| 480 | 98,0 |

### Beispiel 2:

### Extruder 1:

1 kg einer Mischung aus 40 Gew.-% Tramadol-HCl, 15 Gew.-% Ethylcellulose, 35 Gew.-% Isomalt, 7 Gew.-% Polyvinylpyrrolidon K30 und 3 Gew.-% hydriertem Rizinusöl wurden in einem Doppelschneckextrudervermischt.
Temperatur der Schüsse: 44, 80, 121, 99, 92, 82 °C
Düse: 80°C

### Extruder 2:

1 kg einer Mischung aus 52 Gew-% Tramadol-HCL, 36 Gew.-% Hydroxypropylmethylcellulose und 12 Gew.-% Hydroxypropylcellulose wurden in einem Doppelschneckenextruder vermischt.
Temperatur der Schüsse: 43, 78, 123, 124, 131, 131 °C
Düse: 131°C

Die Tabletten wiesen folgende in-vitro-Freisetzung auf:

| Zeit in Minuten | Wirkstoff in Gew.-% |
|---|---|
| 60 | 58,5 |
| 120 | 70,4 |
| 180 | 81,3 |
| 240 | 88,8 |
| 300 | 93,8 |
| 360 | 96,2 |
| 420 | 97,0 |
| 480 | 97,5 |

Die Freisetzungsrate wurde nach dem Ph.Eur.Paddle-Modell in 0,1 N HCl bei 100 U/min und 37 °C und UV-Detektion bei 270 nm ermittelt.

## Patentansprüche

1. Feste, mindestens zweiphasige Zubereitungsformen mindestens eines Opioid-Analgeticums in einer Matrix,
wobei jede der beiden Phasen mindestens ein Opioid enthält, mit einer in-vitro-Freisetzungsrate nach dem Ph. Eur. Paddle Modell von mehr als 50 Gew.-% Wirkstoff nach einer Stunde.

2. Zubereitungsformen nach Anspruch 1, mit einer Freisetzungsrate von 55-80 Gew.-% Wirkstoff nach einer Stunde, 58 - 85 Gew.-% nach zwei Stunden, 60-95 Gew.-% nach vier Stunden und 65-100 gew.-% nach acht Stunden.

3. Zubereitungsformen nach Anspruch 1 oder 2, enthaltend eine schnellfreisetzende Phase und eine retardierte Phase.

4. Zubereitungsformen nach einem der Ansprüche 1 bis 3, enthaltend als Opioid Hydromorphon, Morphin, Tramadol oder deren physiologisch verträgliche Salze.

5. Verfahren zur Herstellung von Zubereitungsformen nach einem der Ansprüche 1 bis 4, dadurch gekennzeichnet, daß die Inhalstsstoffe jeder der Phasen separat in einem Extruder aufgeschmolzen, coextrudiert und zu Arzneiformen geformt werden.
